# EUROPEAN PATENT SPECIFICATION

(11) **EP 2 223 249 B1**
(45) Date of publication and mention of the grant of the patent: **06.11.2019**
(21) Application number: 08851398.1
(22) Date of filing: 25.11.2008
(51) Int. Cl.: G16C 20/50, G16B 15/30

(54) **COMPUTER-BASED MODELING AND DESIGNING OF PHOSPHOFRUCTOKINASE 1 (PFK) MODULATORS**
COMPUTERBASIERTE MODELLIERUNG UND ENTWURFVON MODULATOREN DER PHOSPHOFRUCTOKINASE 1 (PFK)
MODÉLISATION ET CONCEPTION DE MODULATEURS DE PHOSPHOFRUCTOKINASE 1 (PFK) À BASE D'ORDINATEUR

(30) Priority: 25.11.2007 PL 38386807
(43) Date of publication of application: 01.09.2010
(73) Proprietor: Instytut Chemii Bioorganicznej PAN, 61-704 Poznan (PL)
(72) Inventor: RYPNIEWSKI, Wojciech, 60-461 Poznan (PL); BANASZAK, Katarzyna, 61-245 Poznan (PL); ATTINOST, Ingrid, Oro Valley, AZ 85755 (US)
(74) Representative: Witek, Rafal
(86) International application number: PCT/PL2008/000087
(87) International publication number: WO 2009/067033

(56) References cited:
- US-A1- 2007 099 851
- RYPNIEWSKI W. ET AL: "the Structure of Yeast Phosphofructokinase 1" ACTA CRYSTALLOGRAPHICA SECTION A, vol. A61, August 2005 (2005-08), pages C66-C67, XP002521882
- MAGEN A ET AL: "Effect of insulin-induced hypoglycemia on cytoskeleton-bound and cytosolic phosphofructokinase and the levels of glucose 1,6-bisphosphate in rat brain." BIOCHEMICAL AND MOLECULAR MEDICINE DEC 1995 LNKD- PUBMED:8825070, vol. 56, no. 2, December 1995 (1995-12), pages 94-98, XP002579455 ISSN: 1077-3150
- KAWAGUCHI T ET AL: "Regulation of energy metabolism in macrophages during hypoxia. Roles of fructose 2,6-bisphosphate and ribose 1,5-bisphosphate." THE JOURNAL OF BIOLOGICAL CHEMISTRY 27 JUL 2001 LNKD- PUBMED:11373280, vol. 276, no. 30, 27 July 2001 (2001-07-27), pages 28554-28561, XP002579456 ISSN: 0021-9258
- DATABASE CA [Online] CHEMICAL ABSTRACTS SERVICE, COLUMBUS, OHIO, US; May 1997 (1997-05), KASPRZYK, ANNA ET AL: "Regulation of phosphofructokinase activity by fructose 2,6-bisphosphate in insect fat body" XP002579457 retrieved from STN Database accession no. 1997:325955
- DATABASE WPI Week 199038 Thomson Scientific, London, GB; AN 1990-287115 XP002579458 & JP 02 202822 A (KAO CORP) 10 August 1990 (1990-08-10)
- RIDER, MARK H. ET AL: "Fructose 2,6-bisphosphate and its phosphorothioate analog. Comparison of their hydrolysis and action on glycolytic and gluconeogenic enzymes" BIOCHEMICAL JOURNAL , 253(2), 597-601 CODEN: BIJOAK; ISSN: 0306-3275, 1988, XP002579459

## Description

The subject matters of the invention are a computer-based method of designing a PFK modulator and a computer-based method of drug design.

Glycolysis is the basis of anaerobic and aerobic metabolism processes and occurs in almost all organisms (Fothergill-Gilmore & Michels, 1993). It is the main energy source in many prokaryotes and in the eukaryotic cell types devoid of mitochondria or functioning under low oxygen or anaerobic conditions. During glycolysis one glucose molecule is converted to two molecules of pyruvate while two molecules of ATP are produced. The rate of glycolysis is tightly regulated depending on the cell's needs for energy and building blocks for biosynthetic reactions.

Phosphofructokinase (PFK, EC 2.7.1.11) is the main control point in the glycolytic pathway. PFK catalyses the conversion of fructose-6-phosphate (Fru-6-P) to fructose-1,6-diphosphate (Fru-1,6-P₂) with the simultaneous conversion of ATP to ADP. The reaction relases much energy, therefore it is practically irreversible. The reverse reaction is catalysed by a fructose-1,6 bisphosphatase (Benkovic & de Maine, 1982).

PFK is the enzyme with the most complex regulatory mechanism in the glycolytic pathway. The major isozyme of PFK is PFK1, a multi-subunit oligomeric allosteric enzyme whose activity is modulated by a number of effectors. In general, PFK is sensitive to the "energy level" of the cell, as indicated by the levels of ATP relative to the products of ATP hydrolysis, but the mechanisms of control are different in eukaryotes and prokaryotes. In the relatively well studied prokaryotes PFK is activated in response to "low energy level", i.e. by the products of ATP hydrolysis, while in eukaryotes PFK is inhibited by ATP ("high energy") and activated by AMP and, to a lesser extent, by ADP (Hofmann & Kopperschläger, 1982). In a classic case of feedback inhibition PFKs are also inhibited by citrate, allowing feedback from the citric acid cycle (Sols, 1981). In eukaryotes, but not in prokaryotes, PFKs are also activated by fructose-2,6-diphosphate (Fru-2,6-P₂). This potent allosteric regulator, which also inhibits the corresponding bisphosphatase, reflects a higher level of complexity of eukaryotes, compared to prokaryotes. It overrides the inhibition by ATP, which is essential in some tissues (e.g. in muscles), and makes PFK sensitive to the action of the hormones: glucagon and insulin in "higher" organisms (Pilkis et al.,1988; Okar & Lange, 1999).

PFK1 is an allosteric enzyme, showing a characteristic sigmoidal activity profile instead of the common Michaelis-Menten kinetics. The sigmoidal profile indicates co-operativity between the active sites in the oligomeric enzyme. At low substrate concentrations the enzyme shows little activity resulting from its low affinity for the substrate. As the concentration of the substrate increases, so does the substrate affinity and the enzymatic activity. This behaviour is explained in terms of a balance between two alternative forms of the enzyme: the inactive "T-state" and the active "R-state". The ground state of the enzyme is the T-state. It predominates in the absence of the allosteric substrates and allosteric activators. These ligands bind only to the R-state. The binding stabilises the active form, thus enabling more substrate molecules to bind to the still vacant binding sites in the oligomeric enzyme. In the overall effect, the allosteric substrate and the allosteric activators shift the balance in solution from the enzyme's inactive ground T-state towards the active R-state.

In the past, crystallographic studies focused on PFK1 from prokaryotes: *E. coli and B. stearothermophilus.* Prokaryotic PFK1 is a homotetramer with a subunit molecular weight of approximately 37 kDa. In the 1980s, the control mechanism of bacterial PFK1 was investigated by means of protein crystallography. The T- and the R-states of the *E. coli* and *B. stearothermophilus* enzymes were explained in terms of the proteins' quaternary structure. The transitions between the T- and R-states involves a rearrangement of the enzymes' subunits. The binding sites of the substrate, Fru-6-P, and the allosteric effectors span the inter-subunit interface of the R-state. In the T-state
these ligands cannot bind (Evans & Hudson, 1979; Evans et al., 1981; Shirakihara & Evans 1988; Rypniewski & Evans, 1989; Schirmer & Evans, 1990).

The structures of eukaryotic PFK1 are more complex than in prokaryotes. So is their control mechanism. In yeast, PFK1 is an oligomer of the form α₄β₄. Each subunit is more than twice the size of the prokaryotic PFK1 subunit. The amino acid sequence of each eukaryotic subunit consists of two homologous parts, each half being homologous to a prokaryotic PFK1 subunit. The two types of subunits, βα and β, are also homologous in sequence. It has been postulated that yeast PFK1 is the result of two gene duplications: the first tandem duplication resulted in a subunit of double size compared to the prokaryotic subunit, and the second gene duplication created two subunit types (Poorman et al., 1984; Heinisch et al, 1989).

Attempts were made to extend the crystallographic study to include PFK1 in eukaryotes but no suitable crystals could be obtained. Much data were obtained on the eukaryotic PFK by means of biochemistry, enzyme kinetics, genetics, mutagenesis and single-particle electron microscopy but detailed structural information was unavailable.

In the case of PFK1, the allosteric substrate is Fru-6-P (the other substrate, ATP, has no allosteric effect) and there are several allosteric activators of which the most potent is Fru-2,6-P₂, found only in eukaryotes. It abolishes the inhibitory effect of ATP. The Fru-2,6-P₂ binding site, which is part of the regulatory mechanism in eukaryotes, had been proposed, based on amino the acid sequence analysis, which evolved from the active sites that became redundant after the gene duplication and, losing the catalytic function, acquired a regulatory role (Heinisch et al. 1996). However, the Fru-2,6-P₂ binding site has not been described until now in terms of a three-dimensional atomic model.

In the patent application MXPA05011769 (publ. 2006-01-26) crystal of PDE5, its crystalline structure and its use in drug design were presented. The invention relates to the soakable crystals of a phosphodiesterase 5 (PDE5) and their uses in identifying PDE5 ligands, including PDE5 ligands and inhibitor compounds. The present invention also relates to methods of identifying such PDE5 inhibitor compounds and their medical use. The present invention additionally relates to crystals of PDE5 into which ligands may be soaked and crystals of PDE5 10 comprising PDE5 ligands that have been soaked into the crystal.

In the patent application EP 0130030 (publ. 1985-01-02) diagnostic application of phosphofructokinase was described.

In the patent application WO2005083069 (publ. 2005-09-09) PDE2 crystal structures for structure based drug design were described. Crystalline compositions of Phosphodiesterase Type 2 (PDE2), particularly of the PDE2 catalytic domain, and amino acid sequences utilized to form such crystalline compositions, which are used to screen PDE2 ligands. The ligands are formulated into pharmaceutical compositions, and used for treatment of disease states or disorders mediated by PDE2.

In the patent application US2006110743 (publ. 2006-05-25) drug evolution: drug design at "hot spots" was described. A new method of designing and generating compounds having an increased probability of being drugs, drug candidates, or biologically active compounds, in particular having a therapeutic utility, is disclosed. The method consists of identifying a group of bioactive compounds, preferably of diverse therapeutic uses or biological activities and built on a common building block. In this group of compounds, side chains modifying the building block are identified and used to generate a second set of compounds according to the proposed methods of "hybridization", "single substitution" or "incorporation of frequently used side chains". If the compounds in the second set built on the same building block contain an unusually large number of drugs, preferably with diverse therapeutic uses or biological activities, they constitute a "hot spot". A focused combinatorial library of the "hot spot" is then generated, preferably by methods of combinatorial chemistry, and compounds of this library are screened for a variety of therapeutic uses or biological activities. The method generates drugs, drug candidates, or biologically active compounds with a high probability, without requiring any prior knowledge of biological targets.

Despite the above described compounds and methods of designing and generating drugs, drug candidates or biologically active compounds, methods for identifying modulators for metabolic pathways, comprising screening for agents that modulate the activity of enzymes, computer-assisted methods of structure based drug design of different inhibitors using a 3-D structure of a peptide substrates, there is still a need for a successful design of an efficient activator or inhibitor which could therefore exploit the active site's possibilities to accommodate and bind phosphate or other moieties with similar binding potential corresponding to positions 1, 2 and 6 of the sugar ring or moieties corresponding with positions of moieties of fructose ring, which interact with the effector binding site of PFK. In publication Rypniewski et al., The structure of yeast phosphofructokinase 1, Acta Crystallographica A, A61, C66-C67, 2005) authors erroneously identified the ligand bound in the effector binding site as Fru-6-P (the substrate of PFK) instead of the true ligand: the effector Fru-2,6-P2. In publication Magen et al., Effect on insulin-induced hypoglycemia on cytoskeleton-bound and cytosolic phosphofructokinase and the levels of glucose 1,6-bisphosphate in rat brain. Biochem Mol Med. 1995 Dec;56(2):94-8.) it is cited glucose 1,6-bisphosphate is a natural metabolite. This compound fulfils the criteria given in the description of a pharmacophore. Similarly, fructose 2,6-bisphosphate (Fru2,6-P2, the primary natural activator of eukaryotic PFK) also fits the criteria. In publication Kawaguchi et al., Regulation of energy metabolism in macrophages during hypoxia. Roles of fructose 2,6-bisphosphate and ribose 1,5-bisphosphate. J Biol Chem. 2001 276:28554-61 cited ribose 1,5-bisphosphate is a natural metabolite of the pentose phosphate pathway. In publication Kasprzyk et al., "Regulation of phosphofructokinase activity by fructose 2,6-bisphosphate in insect fat body" cited fructose 2,6-bisphosphate is the natural activator of eukaryotic PFK, whose interactions with the PFK enzyme were used as the basis of the design of our pharmacophore.. In publication Database WPI Week 199038 Thompson Scientific...) the cited fructose 1,6-diphosphate and fructose 2,6-diphosphate are the natural physiological ligands of PFK. In publication Rider MH, Kuntz DA, Hue L. Fructose 2,6-bisphosphate and its phosphorothioate analogue. Comparison of their hydrolysis and action on glycolytic and gluconeogenic enzymes.Biochem J. 1988 253:597-601. cited fructose 2,6-bisphosphate is the natural activator of eukaryotic PFK. In patent application US Patent 2007/099851 A1 cited pharmacophore comprise "A" that contains at least one -CH2-group followed by one of the following groups: hydroxy, halogen, phosphate, alkyl, alkoxy, amino, azido or alkenyl.

The goal of the present invention is to provide a computer-based method of designing a PFK modulator and a computer-based method of drug design

The implementation of such a stated goal and the solution of problems dealing with the compounds which may be designed in order to bind in the Fru-2,6-P₂ effector site and induce enzymatic activity of PFK and also compounds which may bind in the effector site without inducing enzymatic activity and preventing the natural activator from binding, and the atomic model which enables the design of both - artificial activators and the binding site blockers ("anti-activators") have been achieved in the present invention.

It is disclosed a crystallographic model of the binding site, being a part of the eukaryotic phosphofructokinase (PFK), in complex with the allosteric activator D-fructose-2,6-bisphosphate (Fru-2,6-P₂), wherein the atomic coordinates x, y, z of a portion of PFK which determine two homologous binding sites of the activator (effector), including the bound Fru-2,6-P₂ molecules, are presented in Tables 1a and 1b, or a derivative set of transformed coordinates expressed in any reference system.

The amino acid residues from Tables 1a or 1b are substituted with the amino acid residues present in a homologous sequence of another eukaryotic PFK.

The three-dimensional structure described by means of the atomic coordinates x, y, z, after being superimposed with the least square minimization method, having the root mean square deviation equal or less than 0.1 nm, in relation to the atomic coordinates x, y, z presented in Tables 1a and 1b.

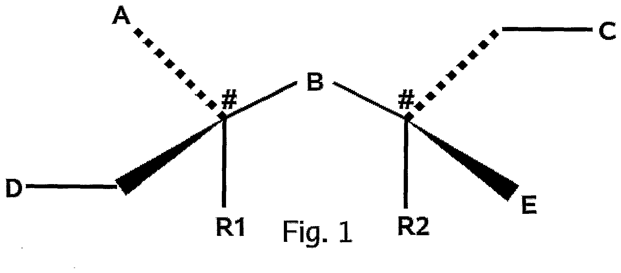

It is presented a modulator which regulates the catalytic activity of PFK, wherein said modulator is a compound presented on Fig. 1, where A and C are selected from among the groups: -PO₄, -SO₄ or -C- SO₂O⁻, and in case of the inhibitor C is -H, B is one of the bridges: -O- or -S-; D is selected from among the groups -PO₄, -SO₄, -OH or -C-S SO₂O⁻, E is -H, # is a C atom with sp³ hybridization; R1 and R2 are either -CXH-OH or -CX=O or -H, where X is a hydrogen atom or bonds with other R groups or bonds with other R groups through the -CH₂- group; and the -CH₂- groups are between D and # and between C and #.

The modulator stimulates the catalytic activity of PFK.

The modulator inhibits the catalytic activity of phosphofructokinasePFK.

The subject of the invention is a computer-based method of designing a PFK modulator, wherein the modulator is a compound of the formula I 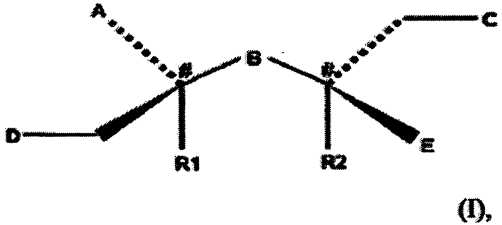 where A and C are selected from among the groups: -PO₄, -SO₄ or -CH₂- SO₂O⁻; and in case of the inhibitor C is -H; B is one of the bridges -O- or -S-; D is selected from among the groups -PO₄, -SO₄, -OH or -CH₂-SO₂O⁻; E is -H, # is a C atom with sp³ hybridization; R1 and R2 are either -CXH-OH or -CX=O or -H, where X is a hydrogen atom or bonds with other R groups or bonds with other R groups through the -CH₂- group; and the -CH₂-groups are between D and # and between C and #. whereinmodulator design includes:
a) exploring the PFK atomic coordinates which constitute the binding site of the PKF effector, presented in Tables 1a or 1b to obtain information about the three-dimensional structure and electrostatic properties of the protein surface;
b) designing a PFK modulator using the effector binding site information given in Tables 1a or 1b.

It is presented a method of selecting the PFK modulator, wherein the modulator is a compound of the formula presented in Fig. 1, and where A and C are selected from among the groups: -PO₄, -SO₄ or -C- SO₂O⁻, and in case of the inhibitor C is -H, B is one of the bridges: -O- or -S-; D is selected from among the groups -PO₄, -SO₄, -OH or -C- SO₂O⁻; E is -H, # is a C atom with sp³ hybridization, R1 and R2 are either -CXH-OH or -CX=O or - H, where X is a hydrogen atom or bonds with other R groups or bonds with other R groups through the -CH₂- group; and the -CH₂- groups are between D and # and between C and #. The modulator design includes:
a) exploring the PFK atomic coordinates which constitute the binding site of the PKF effector, presented in Tables 1a or 1b to obtain information about the structure and properties of the protein surface;
b) selecting a PFK modulator using the effector binding site information given in Tables 1a or 1b.

It is presented a method of producing the PFK modulator comprising the identification of a compound or designing a compound that fits into the effector site binding pocket of PFK in its uninhibited conformation, wherein said conformation of the effector site binding pocket of PFK is defined by the x, y, z coordinates of atoms in the set of amino acid residues given in Tables 1a or 1b.

It is disclosed a computer-based method for the analysis of the interactions of a molecular structure with PFK, which comprises:
a) providing a structure comprising a three-dimensional representation of PFK effector binding site, whose representation comprises all or a portion of the coordinates presented in Tables 1a or 1b, or coordinates whose differences from those are within a root-mean-square deviation (r.m.s.d.) equal or less than 0.1 nm,
b) providing a molecular structure to be fitted to said PFK surface effector binding site; and
c) fitting the molecular structure to the PFK structure of a).
Itis presented a computer-based method for the analysis of molecular structures which comprises:
a) providing the coordinates of at least two atoms of the PFK structure as defined in Tables 1a or 1b, wherein the root mean square deviation for the atoms is equal or less than 0.1 nm ("selected coordinates");
b) providing the structure of a molecular structure to be fitted to the selected coordinates.

The secondsubject of invention is a computer-based method of drug design comprising:
a) providing the coordinates of the PFK structure as defined in Tables 1a or 1b, wherein the root mean square deviation for the atoms is equal or less than 0.1 nm
b) providing the structures of several molecular fragments;
c) fitting the structure of each of the molecular fragments to the providedcoordinates; and
d) assembling the molecular fragments into a single molecule to form a candidate modulator molecule.

It is presented a method of assessing the ability of a candidate modulator to interact in the binding site on the PFK surface, which comprises the steps of:
a) obtaining or synthesising said candidate modulator;
b) forming a crystallized complex of a PFK protein, whose atomic coordinates x, y, z include the coordinates presented in Tables 1a or 1b, or the set of coordinates of a homologous part of protein or candidate modulator expressed in any reference system which, after being superimposed with the least square minimization method, has the root mean square deviation equal or less than 0.1 nm in relation to the atomic coordinates x, y, z presented in Tables 1a or 1b;
c) analysing said complex by means of X-ray crystallography or NMR spectroscopy to determine the ability of said candidate modulator to interact with the binding site on the PFK surface;

It is presented a method of providing data for generating structures and/or designing the drugs which bind the PFK, PFK homologues or analogues, complexes of PFK, or complexes of PFK homologues or analogues with potential modulators, wherein the communication is established with a device which contains the computer-readable data comprising at least one of:
a) atomic coordinate data presented in Tables 1a or 1b, or a set of coordinates expressed in any reference system which, after being superimposed with the least square minimization method, has the root mean square deviation equal or less than 0.1 nm in relation to the atomic coordinates x, y, z presented in Tables 1a or 1b, where such coordinates define the three-dimensional structure of the effector-binding site on the PFK surface;
b) atomic coordinate data of a target effector binding site on the PFK homologue or analogue surface, generated by homology modelling of the target based on the data from Tables 1a or 1b, with the root mean square deviation from atoms equal or less than 0.1 nm;
c) receiving said computer-readable data from a remote device.

It is disclosed a computer system containing at least one of:
a) atomic coordinate data presented in Tables 1a or 1b, or such data which, after being superimposed with the least squares minimization method, has the root mean square deviation from the atoms in Tables 1a or 1b equal or less than 0.1 nm, where such coordinates define the three-dimensional structure of the effector-binding site on the PFK surface or at least its selected coordinates;
b) atomic coordinate data of an effector binding site on the surface of the target PFK protein, generated by homology modelling of the target based on the coordinates from Tables 1a or 1b, where the root mean square deviation from atoms from Tables 1a or 1b, after being superimposed with the least squares minimization method, is equal or less than 0.1 nm;
c) atomic coordinate data of an effector binding site on the surface of the target PFK protein, generated by interpretation of data obtained from the analysis of the X-ray crystallography or NMR, based on the coordinates from Tables 1a or 1b, when the root mean square deviation from atoms from Tables 1a or 1b, after being superimposed with the least squares minimization method, is equal or less than 0.1 nm, and/or
d) crystallographic structure factor data, obtained from the atomic coordinates (c) or (d).

The attached figures facilitate a better understanding of the nature of the present invention.
Figure 1 presents a scheme of a modulator where A and C are selected from -PO₄, -SO₄ or -CH₂- SO₂O⁻ groups, in the case of inhibitor C is -H, B is selected from -O- or -S-bridge, D is selected from -PO₄, -SO₄, -OH or -CH₂- SO₂O⁻ group, E is -H, # is atom C with hybridization sp³, R1 and R2 are selected from -CXH-OH or -CX=O or -H, where X is a hydrogen atom or a bond to the other R-group or a bond to the other R-group via a - CH2- group. Especially important for the modulator is group C. In case of the activator C is sulphate, phosphate or sulphonic group and interacts with neighboring subunit PFK stabilizing the enzyme in active R-state. In case of the inhibitor group C is -H (hydrogen atom) and the modulator does not stabilize the mentioned R-state.
**Figure 2** **and** **Figure 3** show a representative structures of the effector-binding site in α chain (Fig. 2) and β chain (Fig. 3) These chains in the PFK enzyme are similar but not identical. The PFK molecule is a heterooctamer α₄β₄. Bound effector molecules, fructose-2,6-bisphospate (FDP-5, FDP-2) are also depicted.
**Figure 4** **and** **Figure 5** are similar to Figures 2 and 3 but they additionally show a rendering of the molecular surface to illustrate the shape of the effector binding cavity.
**Figure 6** is a scheme of interactions within the effector binding site, between the PFK and the fructose-2,6-bisphosphate effector. Amino acid residues from the α chain are indicated on a white background while residues from the β chain are shown on a grey background.
**Table 1a and 1b** show atomic coordinates in PDB (Protein Data Bank) format of parts of crystallographic atomic PFK from S. cerevisiae (yeast), which are the effector sites on the protein surface, and associated molecules of the effector Fru-2,6-P₂ Table la shows the location of the α-chain on the surface and Table 1b shows the appropriate site for the β-chain. These coordinates are empirically defined as a result of a crystallographic analysis and are a starting point for designing the modulator of the enzymatic activity PFK whose characteristics were described in Fig. 1.

Below, there are example embodiments of the present invention defined above.

### Examples

### Determining the crystal structure of PFK1 in complex with fructose-6-phosphate and fructose-2,6-bisphosphate

PFK from yeast (Saccharomyces cerevisiae) was based on the method of Hofmann & Kopperschläger (1982). Native form of the enzyme - 21S after limited proteolysis by action of α-chymotrypsin at a ratio of 1:600 to PFK, achieved in the presence of 5mM ATP. The tetrameric form of the enzyme 12S created during the digestion was then precipitated with ammonium sulphate (AS), the pellet dissolved, dialysed and loaded on a HPLC Resource Q column twice, to remove the ATP and low molecular weight proteolytic fragments. The purified protein was dialysed at 277 K against 20 mM HEPES buffer, pH 7.4, containing 1 mM EDTA, 0.2 M sodium acetate, 0.1 M ammonium sulphate, 2 mM dithiotreitol (DTT), 0.1 mM phenylmethyl sufonyl fluoride (PMSF) and 10 mM fructose 6-phosphate (Fru-2-P). The crystals were grown by vapour diffusion in hanging drop at 277 K with a reservoir solution containing 6-10 % PEG4000, 0.2 mM sodium acetate in 0.1 M MES buffer, pH 6,0. The protein solution (3 ml, 8 mg/ml) was mixed with an equal volume of the reservoir solution. Crystals in the form of long needles with a diameter 0.2 mm appeared within two weeks.

The crystallographic data were recorded using synchrotron radiation from the crystal under cryo-conditions at 100 K and stabilized with a solution comprises glycerol in concentration 20% (by volume), the reservoir solution and ligands Fru-6-P i Fru-2,6-P₂.

Crystallographic data were processed with the HKL package (Otwinowski and Minor, 1997). Crystals belonged to spatial group P2(1)2(1)2(1) and the unit cell was: *a*=*18,0 nm,* *b*=*18,6 nm, c*=*23,7 nm.* The crystal structure was solved by molecular replacement (MR) using the PFK ttetramer from *E. Coli* as the search model (Shirakihara et al., 1988, PDB code 1 pfk). In the solution of the crystal structure the information concerning the shape of the molecule was also important, it was obtained from electron microscopy (Ruiz et al., 2001). The calculations were carried out using the AmoRe program (Navaza, 1994). The model obtained via the MR method consisted of four tetrameric molecules of *E. coli* PFK. This model was used to calculate the phases. These phases with experimentally determined amplitudes of X-ray scattering structure factors were used to calculate an electron density map. The map was modified with the DM program (Cowtan, 1994) in a procedure that included solvent flattening, histogram matching and non-crystallographic symmetry (NCS) averaging.

The resulting map significantly differed from the initial one which had systematic errors caused by the inaccurate initial model. Phases and resolution were gradually extended in the course of numerous DM cycles, from the initial values of 0.15 - 0.04 nm to the final range of 0.35 - 0.29. The proof of correctness of the performed phase refinement procedures was the fact that the obtained electron density map included the details which were absent in the initial model, for instance the ligand molecules Fru-6-P and Fru-2,6-P₂, and amino acid residues absent in the PFK molecule from E. coli. Subsequently, the chains with the target eukaryotic sequence (Saccharomyces cerevisiae) were built into the electron density map, and the atomic model was refined with the CNS program (Brunger et al., 1998). The refinement cycles were interspersed with manual model corrections based on the 2Fo-Fc and Fo-Fc maps. The temperature factors were not refined initially, but with time they were refined for individual side chains and separately for the main chain atoms of individual amino acid residues. The statistics, such as R-factor and R-free (based on 5% of reflexes) were monitored during the refinement, as well as the FOM (*figure of merit*) (Brunger, 1992). The final values of those statistics were as follows: R = 0.238, R-free = 0.311, FOM = 0.73. The atomic model was validated with the PROCHECK software (Laskowski et al., 1993). All indices were within tolerance limits, or better than it could be expected for a structure determined at such a resolution. For instance, on the Ramachandran plot 79.0% of the amino acid residues are located in the most favoured region, whereas the expected value for the 0.029 nm resolution is 68.7%. The 2Fo-Fc electron density map allows an unambiguous determination of the course of the polypeptide chains and the correct "register" of sequences, because both the density for the main chain and the typical shapes of side chains made such determination possible. All four independent models of α chains and the β chains are consistent with each other. The parts of the molecule which have their equivalents in the sequence of PFK from E. coli are also consistent in terms of their spatial structure.

The final model includes more than six thousand amino acid residues which constitute eight polypeptide chains (four α and four β), eight Fru-6-P molecules and eight Fru-2,6-P₂ molecules. These chains, situated in the asymmetric unit of the crystallographic unit cell, have a form very similar to the general shape of the molecule as it was determined by electron microscopy (EM), despite the fact that the crystal contains the 12S molecules, that is the form obtained as a result of partial proteolysis (see above) and being tetrameric in solution, while the EM results are for the native octameric 21S form. It is however evident that in the crystalline structure the tetrameric 12S molecules associate in pairs, just like in the native form, and create an octamer in which four α subunits constitute the core of the molecule and the β subunits are on the outside. The structures of α and β chains are similar (just like the amino acid sequences), each of them resembling a dimer of the PFK subunits from E. coli. The α and β chains associate in pairs so that their dimer structure resembles. the tetramer of the PFK subunits from the bacteria. Four such dimers associate and create the octameric PFK molecule from S. cerevisiae. The determined structure corresponds to the active form (R-state) that is the quaternary form of the molecule which allows the binding of the Fru-6-P substrate. The binding of Fru-2,6-P₂ in the effector site is analogous to the binding of Fru-6-P in the substrate binding site. Both these ligands are visible in the crystal structure. The other argument for the R form in this crystalline structure is also the comparison with the structures of bacterial PFK of both forms. The interactions between the subunits in the eukaryote structure are comparable to the active structures of the PFK bacterial forms (R-state), and not to inactive forms (T-state).

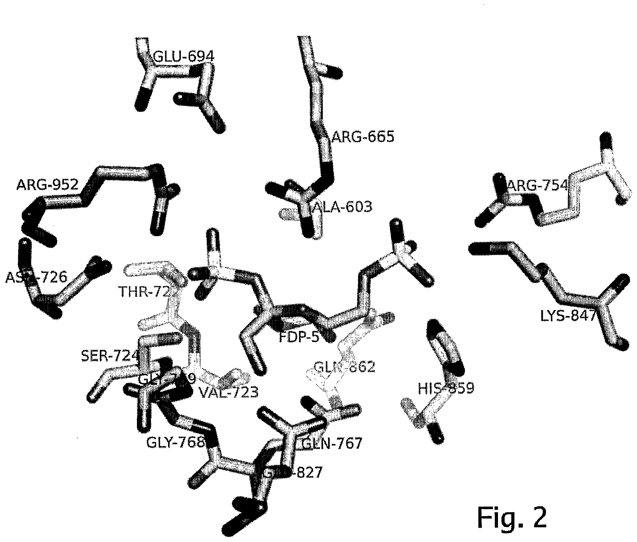

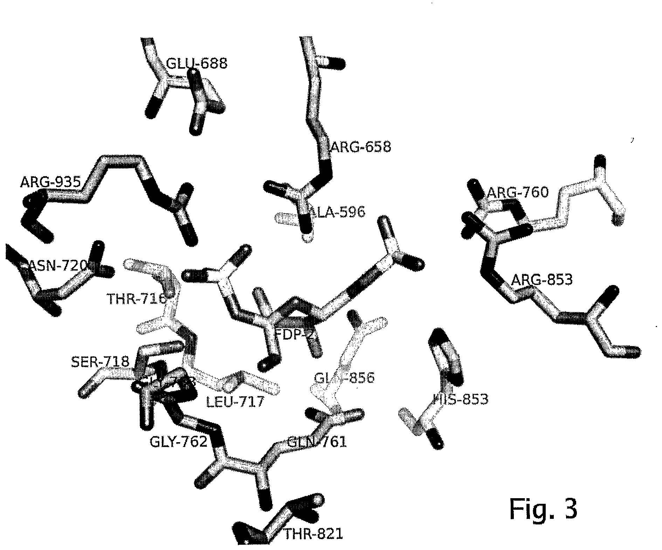

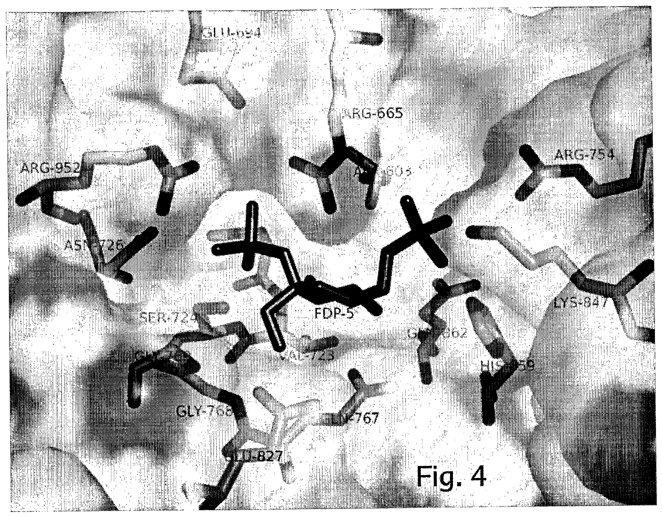

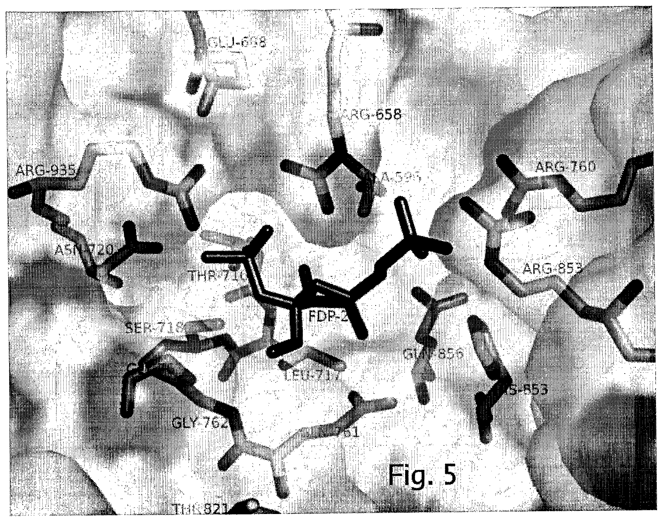

The crystalline structure of PFK from S. cerevisiae is the first structure of eukaryotic PFK regulated by Fru-2,6-P₂ (which is typical of eukaryotes) to have been determined experimentally in terms of a three-dimensional atomic model. The model shows detailed interactions of PFK with the Fru-2,6-P₂ activator and explains the mechanism of its action. The effector binds between two subunits and stabilizes the quaternary structure of the enzyme in the active form (R-state). Such a role of the Fru-2,6-P₂ enzyme was earlier postulated on the basis of evolution consideration, but only here the presented atomic structure of the effector bond site along with the associated Fru-2,6-P₂ molecule allows an understanding of this mechanism in terms of actual interatomic interactions, such as hydrogen bonds or the Van der Waals forces. The accurate determination of the ligand binding site allows also a determination of an optimum match of the ligand molecule in the steric sense. The presented model of the effector-ligand bond site is the key to control the activity of the eukaryotic PFK (Fig. 1 to 6).

The detailed model of the effector binding site of PFK and its interactions with Fru-2,6-P₂ constitute the matrix for structure-based design of compounds different than the native Fru-2,6-P₂ ligand, but sharing with it some common features, which compounds are strong activators or inhibitors of this PFK or related enzymes. It is possible that Fru-1,6-P₂ and Fru-6-P can also bind at the effector binding site. The analysis of the atomic model of the PFK in complex with Fru-2,6-P₂ indicates such a possibility. The PFK surface has a suitable cavity which could accommodate the phosphate group bonded with 1-carbon of the fructose ring. The designing of an artificial effector involves taking advantage of the possibility of fitting and specifally binding appropriate groups in the cavity that constitutes the Fru-2,6-P₂ binding site on the surface of the eukaryotic PFK.

The proposed compounds are designed in analogy to the molecule Fru-2,6-P₂ bound in the effector sites on the PFK surface (Tables 1a and 1b). Such a compound has the ability to bind to PFK in the position corresponding to the phosphate group bound with the C6 atom of the fructose ring, and binding PFK in the position corresponding to the phosphate group at the C2 atom, or in the position corresponding to the substituent at the C1 atom, or in the positions corresponding to the fructose ring groups which are capable of making hydrogen bonds.

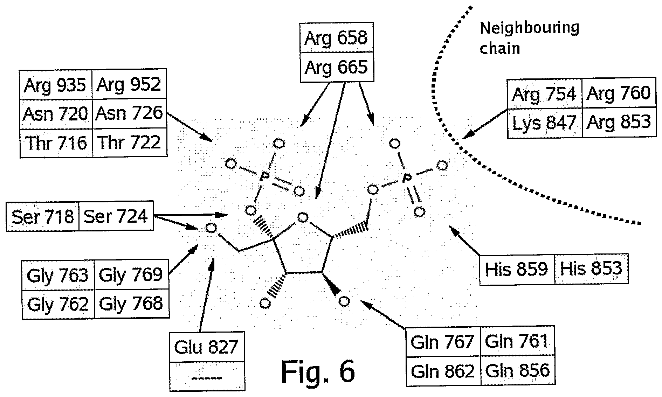

Figure 6 provides a summary of the experimentally determined interactions in the effector site. The analysis of such interactions, with due consideration of steric conditions, has resulted in determining the "active part", or a pharmacofore, of an artificial ligand which would have activator properties and also one which would be a PFK inhibitor. The "active part" is presented in Figure 1. The activator should have as many as possible of the features defined in the presented diagram and description to Fig. 1. The most important for the activator is the presence of the "C" group corresponding to 6-phosphate in the natural activator Fru-2,6-P₂. Whereas the larger part of the ligand is bound to one PFK subunit, the "C" group binds a neighbouring subunit and stabilizes the quaternary structure of the enzyme in the active form (R-state).

In case of the inhibitor, the most important is the absence of the "C" group, or rather its substitution by a hydrogen atom bound with the preceding carbon atom. Then the ligand only blocks the effector binding site which results in the enzyme stabilization in the inactive form (T-state) which is dominant when the activators are absent.

The presented results have been developed based on the examination of the yeast PFK structure, but they can be used for the majority of eukaryotic PFKs which have the mechanism of activation by Fru-2,6-P₂, including the human PFK. PFK is an important point of controlling the metabolism and is subject to a complex process of regulation. The balance between the aerobic and anaerobic metabolism and the balance between the glycolysis and the gluconeogenesis are to a large extent dependent on the PFK activity. Artificial stimulation or inhibition of PFK could disturb this delicate balance, but it could be also be used in medicine. For example, PFK plays an important part in the generation of heat by the organism. Its activity increases when the ambient temperature drops. This is the so-called futile cycle of PFK acting in tandem with a corresponding bisphosphatase. The goal of this process is to generate heat. A synthetic PFK activator as defined in this invention could be used in cases of hypothermia. It is generally known that restoring the correct body temperature in the hypothermic body is not an easy task. More examples could be given where it would be advantageous to stimulate the PFK activity and therefore the glycolytic pathway and related processes. It is possible to use the activator as a drug in case of genetic illnesses related to a low PFK activity. Also a PFK inhibitor could be used in medicine. The inhibitor proposed in this invention is similar to the activator, but it lacks the "C" group (Figure 1) which blocks the effector position without the activation effect.

## Claims

1. A computer-based method of designing a PFK modulator, wherein the modulator is a compound of the formula I 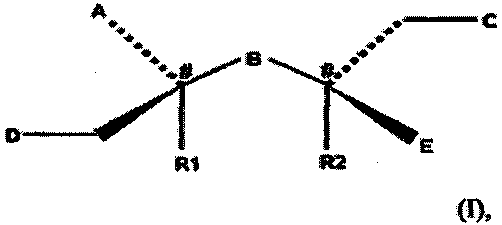
where A and C are selected from among the groups: -PO₄, -SO₄ or -CH₂-SO₂O⁻; and in case of the inhibitor C is -H; B is one of the bridges -O- or -S-; D is selected from among the groups -PO₄, -SO₄, -OH or -CH₂-SO₂O⁻; E is -H, # is a C atom with sp³ hybridization; R1 and R2 are either -CXH-OH or -CX=O or -H where X is a hydrogen atom or bonds with other R groups or bonds with other R groups through the -CH₂ - group; and the -CH₂ - groups are between D and # and between C and #,
wherein the modulator design includes:
a) exploring the PFK atomic coordinates which constitute the binding site of the PKF effector presented in Tables 1a or 1b to obtain information about the three- dimensional structure of the protein surface;
b) designing a PFK modulator using the effector binding site information given in Tables 1a or 1b.

2. A computer-based method of drug design comprising:
a) providing the coordinates of the PFK structure as defined in Tables 1a or 1b, wherein the root mean square deviation for the atoms is equal or less than 0.1 nm;
b) providing the structures of several molecular fragments;
c) fitting the structure of each of the molecular fragments to the provided coordinates; and
d) assembling the molecular fragments into a single molecule to form a candidate modulator molecule.

## Patentansprüche

1. Computergestütztes Verfahren zum Entwerfen eines PFK-Modulators, wobei der Modulator eine Verbindung der Formel I ist: 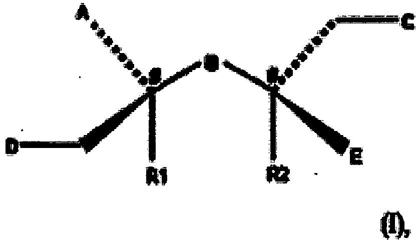
wobei A und C ausgewählt sind aus den Gruppen: -PO₄, -SO₄ oder -CH₂-SO₂O-; und im Falle des Inhibitors C -H ist; B eine der Brücken -O- oder -S- ist; D ausgewählt ist aus den Gruppen -PO₄, -SO₄, -OH oder -CH₂-SO₂O-; E -H ist, # ein C-Atom mit sp³-Hybridisierung ist; R1 und R2 eines von -CXH-OH oder -CX=O oder -H sind, wobei X ein Wasserstoffatom ist oder Bindungen mit anderen R-Gruppen oder Bindungen mit anderen R-Gruppen über die -CH₂-Gruppe sind; und die -CH₂-Gruppen zwischen D und # und zwischen C und # liegen,
wobei der Modulatorentwurf umfasst:
a) Untersuchen der PFK-Atomkoordinaten, die die Bindungsstelle des in den Tabellen 1a oder 1b dargestellten PKF-Effektors bilden, um Informationen über die dreidimensionale Struktur der Proteinoberfläche zu erhalten;
b) Entwerfen eines PFK-Modulators unter Verwendung der in den Tabellen 1a oder 1b gegebenen Effektor-Bindungsstelle-Informationen.

2. Computergestütztes Verfahren zum Wirkstoffentwurf, umfassend:
a) Bereitstellen der Koordinaten der PFK-Struktur, wie in den Tabellen 1a oder 1b definiert, wobei die Standardabweichung für die Atome gleich ist wie oder kleiner ist als 0,1 nm;
b) Bereitstellen der Strukturen mehrerer molekularer Fragmente;
c) Anpassen der Struktur jedes der molekularen Fragmente an die bereitgestellten Koordinaten; und
d) Zusammenfügen der molekularen Fragmente zu einem einzelnen Molekül, um ein Kandidatenmodulatormolekül zu bilden.

## Revendications

1. Procédé assisté par ordinateur pour concevoir un modulateur de PFK, dans lequel le modulateur est un composé de formule I 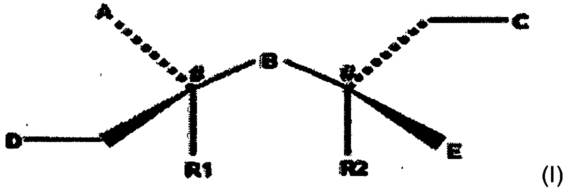
dans laquelle A et C sont choisis parmi les groupes: -PO₄, -SO₄ ou -CH₂-SO₂O⁻ ; et dans le cas où l'inhibiteur C est -H ; B est l'un des ponts -O- ou -S- ; D est choisi parmi les groupes -PO₄, -SO₄, -OH ou -CH₂-SO₂O⁻ ; E est -H, # est un atome C avec hybridation sp³ ; R1 et R2 sont soit -CXH-OH soit - CX-O soit -H lorsque X est un atome d'hydrogène ou se lie à d'autres groupes R ou se lie à d'autres groupes R par l'intermédiaire du groupe -CH₂- ; et les groupes -CH₂- sont entre D et # et entre C et #,
dans lequel la conception de modulateur comprend:
a) l'exploration des coordonnées atomiques de PFK qui constituent le site de liaison de l'effecteur de PFK, présentées dans le Tableau 1a ou 1b, pour que soient obtenues des informations concernant la structure tridimensionnelle de la surface de la protéine;
b) la conception d'un modulateur de PFK utilisant les informations de site de liaison à l'effecteur indiquées dans le Tableau 1a ou 1b.

2. Procédé assisté par ordinateur de conception d'un médicament, comprenant:
a) l'obtention des coordonnées de la structure de PFK telles que définies dans le Tableau 1a ou 1b, l'écart quadratique moyen pour les atomes étant égal ou inférieur à 0,1 nm;
b) l'obtention des structures de plusieurs fragments moléculaires;
c) l'ajustement de la structure de chacun des fragments moléculaires aux coordonnées obtenues; et
d) l'assemblage des fragments moléculaires en une seule molécule pour former une molécule de modulateur candidate.
